# EUROPEAN PATENT SPECIFICATION

(11) **EP 0 595 526 B1**
(45) Date of publication and mention of the grant of the patent: **10.09.1997**
(21) Application number: 93308301.6
(22) Date of filing: 19.10.1993
(51) Int. Cl.: A61K 31/55

(54) **Peripheral circulation improving agent**
Mittel zur Verbesserung der peripheren Durchblutung
Agent améliorant la circulation périphérique

(30) Priority: 23.10.1992 JP 284834/92
(43) Date of publication of application: 04.05.1994
(73) Proprietor: TANABE SEIYAKU CO., LTD., Chuo-ku Osaka (JP)
(72) Inventor: Murata, Sakae, Kawagoe-shi, Saitama-ken (JP); Narita, Hiroshi, Urawa-shi, Saitama-ken (JP); Kaburaki, Minako, Toda-shi, Saitama-ken (JP)
(74) Representative: Baverstock, Michael George Douglas

(56) References cited:
- EP-A- 0 154 838
- EP-A- 0 158 340
- EP-A- 0 318 398
- EP-A- 0 476 854
- EP-A- 0 555 042
- CA-A- 1 291 134
- FR-A- 2 626 000
- Week 8411, Derwent Publications Ltd., London, GB; AN 84-065035; & JP-A-59 020 221

## Description

### BACKGROUND OF THE INVENTION

The present invention relates to a peripheral circulation improving agent. More particularly, the present invention relates to a peripheral circulation improving agent which has an improving activity on peripheral circulation and is useful as a curing and/or prophylactic agent of chronic arterial obstruction and Buerger's disease.

It has been known that 1,5-benzothiazepine derivatives such as 2-(4-lower alkylphenyl)-3-lower alkanoyloxy (or hydroxy)-5-(2-di-lower alkylaminoethyl)-8-lower alkyl-2,3-dihydro-1,5-benzothiazepin-4(5H)-one and 2-(4-lower alkylphenyl)-3-lower alkanoyloxy (or hydroxy)-5-(2-mono-lower alkylaminoethyl)-8-lower alkyl-2,3-dihydro-1,5-benzothiazepin-4(5H)-one have an antihypertensive, cerebral and coronary vasodilating and/or platelet aggregation-inhibiting activities (for example, EP-A-0 154 838 and EP-A-0 158 340).

EP-A-476 854 describes a pharmaceutical composition for inhibiting platelet aggregation comprising acetylsalicylic acid or a pharmaceutically acceptable salt thereof and a 1,5-benzothiazepine derivative.

EP-A-318 398 and JP-A-59 020221 describe pharmaceutical preparations of Diltiazem for use in the treatment of Raynaud's disease and peripheral circulation deficiency.

### SUMMARY OF THE INVENTION

The present invention is to provide a peripheral circulation improving agent.

The inventors of the present invention have found that a (-)-cis isomer of the above 1,5-benzothiazepine derivative has a specific peripheral circulation improving activity and accomplished the present invention.

That is, the present invention relates to a peripheral circulation improving agent comprising, as an active ingredient, the (-)-cis-1,5-benzothiazepine derivative, represented by the following formula: 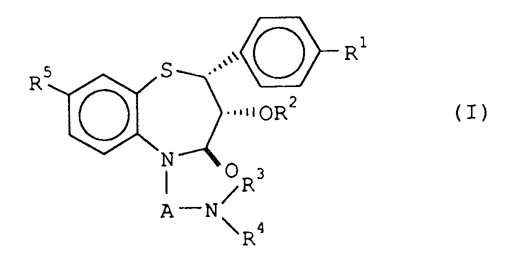 wherein R¹, R³, R⁴ and R⁵ represent a methyl group; R² represents an acetyl group and A represents an ethylene group,
or a pharmaceutically acceptable salt thereof.

According to the present invention there is provided the use of the above (-)-cis-1,5-benzothiazepine derivative or a pharmaceutically acceptable salt thereof in the preparation of pharmaceutical compositions without aspirin which are useful for the prophylaxis or treatment of chronic arterial obstruction and in particular the prophylaxis or treatment of Buerger's disease.

### DESCRIPTION OF THE PREFERRED EMBODIMENT

The (-)-cis-1,5-benzothiazepine derivative (I) and the pharmaceutically acceptable salt thereof, the active component of the present invention have an outstanding advantage that it exhibits a high improving activity on peripheral circulation, i.e., it remarkably increases a femoral arterial blood flow, and that the activity continues for a long period of time.

Furthermore, the (-)-cis-1,5-benzothiazepine derivative (I) has a low toxicity and a high safety. For example, the above described test compound A was orally given to a mouse in a dosage of 1000 mg/kg, no dead case was observed even after 2 days.

Accordingly, the (-)-cis-1,5-benzothiazepine derivative (I) or the pharmaceuticllly acceptable salt thereof of the present invention is useful as a peripheral circulation improving agent, and can be effectively used for curing and/or prophylaxis of chronic arterial obstruction, and Buerger's disease in warm-blooded animals including human beings.

The (-)-cis-1,5-benzothiazepine derivative (I) as the active ingredient of the present invention can be used for a medical use of either in free form or in the form of a pharmaceutically acceptable salt thereof. The pharmaceutically acceptable salt may include, for example, an inorganic acid addition salt such as hydrochloride, hydrobromide, hydroiodide, perchlorate, sulfonate and phosphate; or an organic acid addition salt such as oxalate, maleate, fumarate, tartarate and methanesulfonate.

The dosage of the (-)-cis-1,5-benzothiazepine derivative (I) or the pharmaceutically acceptable salt thereof may vary depending on the age, the body weight and the health condition of patient, or on the severity of disease, but is about 0.1 to 300 mg/kg per day, preferably 1 to 30 mg/kg per day for oral administration; and about 0.001 to 10 mg/kg per day, preferably 0.01 to 0.3 mg/kg per day for parenteral administration.

The (-)-cis-1,5-benzothiazepine derivative (I) or the pharmaceutically acceptable salt thereof can be used by way of either oral administration or parenteral administration.

The (-)-cis-1,5-benzothiazepine derivative (I) or the pharmaceutically acceptable salt thereof can be orally administered in a dosage form of a solid preparation such as tablets, pills, capsules and powder, and a liquid preparation such as solutions and suspensions, together with a pharmaceutical carrier suitable for oral administration as a pharmaceutical preparation. Such a carrier may include, for example, conventional binders (e.g. syrup, gum arabic, gelatin, sorbitol, tragacanth and polyvinylpyrrolidone), excipients (e.g. lactose, sugar, corn starch, potassium phosphate, sorbitol and glycine), lubricants (e.g. magnesium stearate, talc, polyethyleneglycol and silica), disintegrators (e.g. potato starch), or humectants (e.g. sodium lauryl sulfate).

On the other hand, in the case of parenteral administration, the composition may be preferably used as an injection or injection for drip infusion, by using, for example, distilled water for injection, physiological saline and glucose aqueous solution.

The (-)-cis-1,5-benzothiazepine derivative (I) as the active component of the present invention can be prepared according to a conventional method (for example, methods disclosed in U.S. Patent No. 3,562,257, EP-A-0 154 838, EP-A-0 158 340, and Italian Patent No. 1,227,852).

For example, the derivative can be prepared by reacting a compound represented by the formula (II): 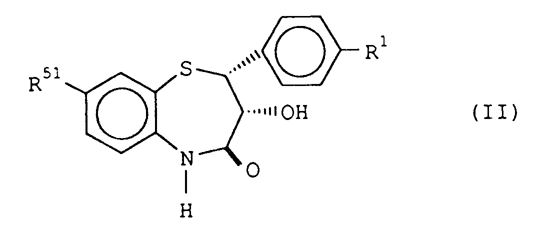 wherein R⁵¹ represents a methyl group; and the other symbols have the same meanings as defined above,
with a compound represented by the formula (III): 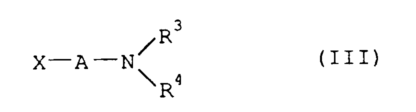 wherein X represents a halogen atom; and the other symbols have the same meanings as defined above,
in the presence of a salt (e.g., an alkali metal salt of carbonic acid), and, if necessary, reacting the resulting compound with an acid anhydride of a compound represented by the formula (IV):

R²¹-OH (IV)

wherein R²¹ represents an acetyl group,
in the presence of a deacidifying agent (e.g., an organic amine).

### EXAMPLES

### Experimental example 1

### (Test Compounds)

The compounds listed in the following Table 1 were used as the test compounds.

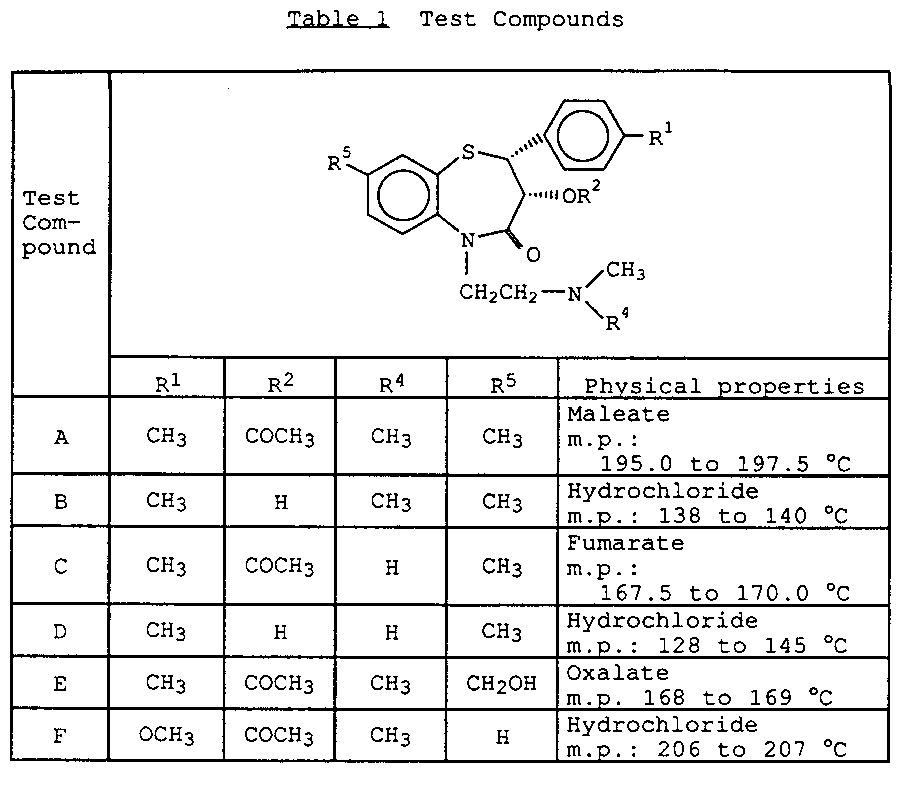

### (Method)

Adult dogs (one group consisting of a dog) were intravenously given the test compound under anesthesia and artificial respiration successively at intervals of 60 minutes so that the dose can be 10, 30, 100 and 300 µg/kg body weight, and the blood flow of femoral artery was measured with a time course. The femoral arterial blood flow was measured with an electromagnetic blood flow meter (manufactured by Nihon Koden, MFV-2100, trade name) through a flow probe attached to the femoral artery. An increase in the femoral arterial blood flow was calculated by subtracting the blood flow before the administration of the first dose of test compound as the standard value (the base line) from that 60 minutes after each administration.

### (Results)

The increase in the femoral arterial blood flow after the administration of the test compound is shown in Table 2.

**Table 2**

| Test Compound | Increase in femoral arterial blood flow (ml/min) | | | |
|---|---|---|---|---|
| | Dosage (µg/kg) | | | |
| | 10 | 30 | 100 | 300 |
| A | 13 | 30 | 34 | 32 |
| B | 22 | 28 | 48 | 74 |
| C | 6 | 19 | 50 | 65 |
| D | - | - | 11 | 35 |
| E | - | 15 | 37 | 36 |
| F | 11 | 21 | 27 | 37 |

### Experimental example 2

### (Method)

The test compound A was intravenously administered to adult dogs (one group consisting of 4 dogs) in a dosage of 100 µg/kg body weight and the femoral arterial blood flow was measured at 30, 60, 120, 180, 240 and 300 minutes after the administration in the same manner as in Experimental example 1 to determine an increase in the blood flow based on that before the administration of the test compound.

### (Results)

The femoral arterial blood flow and the increase in the blood flow after the administration of the test compound A are shown in Table 3.

**Table 3**

| | Period after administration (min) | | | | | | |
|---|---|---|---|---|---|---|---|
| | Before | 30 | 60 | 120 | 180 | 240 | 300 |
| Blood flow (ml/min) | 80.8 | 136.3 | 146.3 | 143.5 | 141.3 | 141.5 | 136.3 |
| Increase in blood flow (ml/min) | | 55.5 | 65.5 | 62.8 | 60.5 | 60.8 | 55.5 |

### Experimental example 3

### (Test Compounds)

The compounds A, B, C, E and F listed in Table 1 were used as the test compounds.

### (Method)

Adult dogs (one group consisting of 5 dogs) were intravenously given the test compound under anesthesia and artificial respiration successively at intervals of 60 minutes so that the dose can be 10, 30, 100, 300 and 1000 µg/kg body weight, and the blood flow of femoral artery was measured with a time course. The femoral arterial blood flow was measured with an electromagnetic blood flow meter (manufactured by Nihon Koden, MFV-2100, trade name) through a flow probe attached to the femoral artery. An increase in the femoral arterial blood flow was calculated by subtracting the blood flow before the administration of the first dose of test compound as the standard value (the base line) from that of maximum during an interval of 60 minutes after each administration.

### (Results)

The increase in the femoral arterial blood flow after the administration of the test compound is shown in Table 4.

**Table 4**

| Test Compound | Increase in femoral arterial blood flow (ml/min) | | | | |
|---|---|---|---|---|---|
| | Dosage (µg/kg) | | | | |
| | 10 | 30 | 100 | 300 | 1000 |
| A | 5.6 | 40.4 | 55.2 | 61.4 | |
| B | 10.4 | 16.4 | 41.4 | 70.2 | 79.2 |
| C | - | 6.8 | 25.6 | 44.6 | 49.0 |
| E | - | 15.6 | 55.6 | 69.2 | 68.6 |
| F | 10.7 | 21.0 | 27.3 | 37.0 | |

### Preparation example

(1) A mixture comprising 2.48 g of (-)-cis-2-(4-methylphenyl)-3-hydroxy-8-trityloxymethyl-2,3-dihydro-1,5-benzothiazepin-4(5H)-one, 0.75 g of 2-(dimethylamino)ethyl chloride hydrochloride, 1.70 g of potassium carbonate and 50 ml of acetone was refluxed overnight while stirring. After the mixture was cooled, insoluble materials were removed by filtration, and the filtrate was concentrated under reduced pressure. The residual was recrystallized from a mixed solution of ethyl acetate and n-hexane to give 2.59 g of (-)-cis-2-(4-methylphenyl)-3-hydroxy-5-[2-(dimethylamino)ethyl]-8-trityloxymethyl-2,3-dihydro-1,5-benzothiazepin-4(5H)-one.
   m.p.: 110 to 114 °C
   [α]_{D}²⁰ -83.7° (c = 0.251, methanol).
(2) A mixture of 2.46 g of the compound obtained in the above (1), 9 ml of acetic anhydride and 25 ml of pyridine was stirred overnight at room temperature. The reaction mixture was poured into water with ice, and extracted with ethyl acetate. The extract was washed, dried, and concentrated under reduced pressure to give 2.72 g of (-)-cis-2-(4-methylphenyl)-3-acetoxy-5-[2-(dimethylamino)ethyl]-8-trityloxymethyl-2,3-dihydro-1.5-benzothiazepin-4(5H)-one as an oily material.
   IR^{liquid} νₘₐₓ (cm⁻¹): 1745, 1680.
(3) The compound obtained in the above (2) in an amount of 2.0 g was dissolved in 20 ml of trifluoroacetic acid, followed by stirring at room temperature for 3 hours, and the mixture was concentrated under reduced pressure. To the residue were added 200 ml of saturated aqueous solution of sodium hydrogen carbonate and 100 ml of diethyl ether and the resulting mixture was stirred at room temperature for 30 minutes.

The reaction mixture was extracted with ethyl acetate, and the extract was concentrated under reduced pressure to give (-)-cis-2-(4-methylphenyl)-3-acetoxy-5-[2-(dimethylamino)-ethyl]-8-hydroxymethyl-2,3-dihydro-1,5-benzothiazepin-4(5H)-one as a crude product.

The product was treated with oxalic acid, and recrystallized from a mixed solution comprising ethanol and ether to give 966 mg of (-)-cis-2-(4-methylphenyl)-3-acetoxy-5-[2-(dimethylamino)ethyl]-8-hydroxymethyl-2,3-dihydro-1,5-benzothiazepin-4(5H)-one oxalate.
m.p.: 168 to 169 °C
[α]_{D}²⁰ -83.1° (c = 0.201, methanol).

## Claims

1. The use of a (-)-cis-1,5-benzothiazepine compound represented by the following formula: 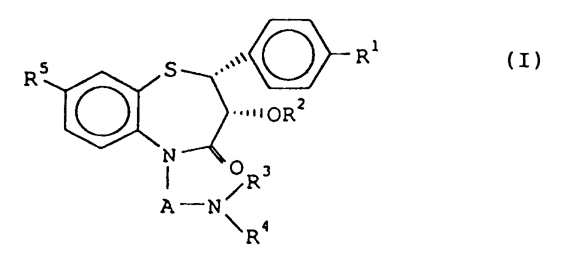 wherein R¹, R³, R⁴, and R⁵ represent a methyl group, R² represents an acetyl group and A represents an ethylene group,
or a pharmaceutically acceptable salt thereof in the preparation of a pharmaceutical composition without aspirin for the prophylaxis or treatment of chronic arterial obstruction.

2. The use of a (-)-cis-1,5-benzothiazepine compound represented by the following formula: 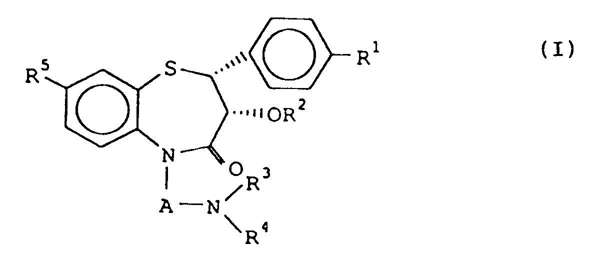 wherein R¹, R³, R⁴, and R⁵ represent a methyl group, R² represents an acetyl group and A represents an ethylene group,
or a pharmaceutically acceptable salt thereof in the preparation of a pharmaceutical composition without aspirin for the prophylaxis or treatment of Buerger's disease.

## Patentansprüche

1. Verwendung einer (-)-Cis-1,5-benzothiazepin-Verbindung, dargestellt durch die folgende Formel: 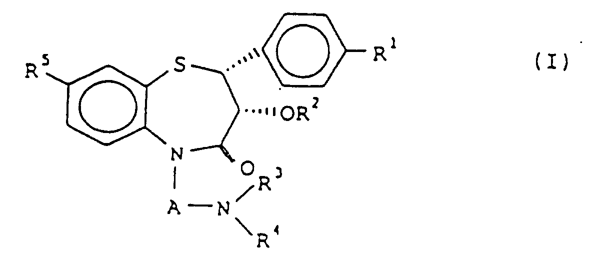 worin R¹, R³, R⁴ und R⁵ eine Methylgruppe darstellen, R² eine Acetylgruppe darstellt und A eine Ethylengruppe darstellt,
oder eines pharmazeutisch verträglichen Salzes davon bei der Herstellung einer pharmazeutischen Zusammensetzung ohne Aspirin für die Prophylaxe oder zur Behandlung von chronischer Arterienverstopfung.

2. Verwendung einer (-)-Cis-1,5-benzothiazepin-Verbindung, dargestellt durch die folgende Formel: 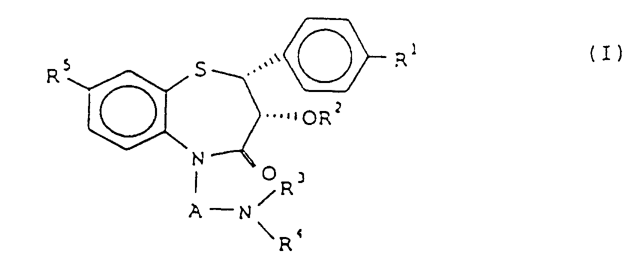 worin R¹, R³, R⁴ und R⁵ eine Methylgruppe darstellen, R² eine Acetylgruppe darstellt und A eine Ethylengruppe darstellt,
oder eines pharmazeutisch verträglichen Salzes davon bei der Herstellung einer pharmazeutischen Zusammensetzung ohne Aspirin für die Prophylaxe oder zur Behandlung von chronischer Buerger's Krankeit.

## Revendications

1. Utilisation d'un composé (-)-cis-1,5-benzothiazépine représenté par la formule générale: 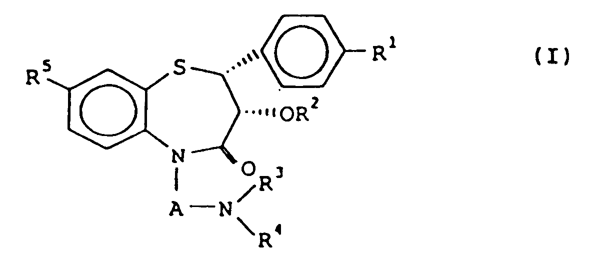 dans laquelle R¹, R³, R⁴ et R⁵ représentent un groupe méthyle, R² représente un groupe acétyle et A représente un groupe éthylène,
ou d'un de ses sels pharmaceutiquement acceptables, dans la préparation d'une composition pharmaceutique sans aspirine pour la prophylaxie ou le traitement de l'obstruction artérielle chronique.

2. Utilisation d'un composé (-)-cis-1,5-benzothiazépine représenté par la formule générale: 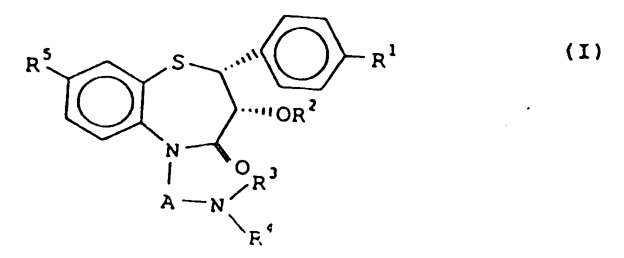 dans laquelle R¹, R³, R⁴ et R⁵ représentent un groupe méthyle, R² représente un groupe acétyle et A représente un groupe éthylène,
ou d'un de ses sels pharmaceutiquement acceptables, dans la préparation d'une composition pharmaceutique sans aspirine pour la prophylaxie ou le traitement de la maladie de Buerger.
